# FASCICULE DE BREVET EUROPEEN

(11) **EP 2 709 994 B1**
(45) Date de publication et mention de la délivrance du brevet: **25.07.2018**
(21) Numéro de dépôt: 12723154.6
(22) Date de dépôt: 16.05.2012
(51) Int. Cl.: C07D 307/48, C07D 307/46, C07D 307/50

(54) **PROCÉDÉ DE PRÉPARATION DE 5-HYDROXYMÉTHYLFURFURAL**
VERFAHREN ZUR HERSTELLUNG VON 5-HYDROXYMETHYLFURFURAL
PROCESS FOR THE PREPARATION OF 5-HYDROXYMETHYLFURFURAL

(30) Priorité: 16.05.2011 FR 1154232
(43) Date de publication de la demande: 26.03.2014
(73) Titulaire: CENTRE NATIONAL DE LA RECHERCHE SCIENTIFIQUE (C.N.R.S.), 75016 Paris (FR); UNIVERSITE CLAUDE BERNARD - LYON 1, 69100 Villeurbanne (FR)
(72) Inventeur: ESSAYEM, Nadine, F-38540 Saint Just Chaleyssin (FR); LOPES DE SOUZA, Rodrigo, Rio de Janeiro (BR); RATABOUL, Franck, F-69008 Lyon (FR)
(74) Mandataire: Lavoix
(86) Numéro de dépôt international: PCT/EP2012/059193
(87) Numéro de publication internationale: WO 2012/156479

(56) Documents cités:
- EP-A2- 0 230 250
- WO-A1-2007/146636
- CN-A- 101 381 351
- GB-A- 600 871
- US-A- 2 929 823
- ANTAL M. J. ET AL.: "Mechanism and formation of 5-(hydroxymethyl)-2-furaldehyde from D-fructose and sucrose", CARBOHYDRATE RESEARCH, vol. 199, 1990, pages 91-109, XP002657310, cité dans la demande
- HAWORTH W. N. AND JONES W. G. M.: "The Conversion of Sucrose into Furan Compounds. Part I. 5-Hydroxymethylfurfuraldehyde and Some Derivatives", JOURNAL OF THE CHEMICAL SOCIETY, 1944, pages 667-670, XP009151383,
- CHAREONLIMKUN A ET AL: "Reactions of C5 and C6-sugars, cellulose, and lignocellulose under hot compressed water (HCW) in the presence of heterogeneous acid catalysts", FUEL, IPC SCIENCE AND TECHNOLOGY PRESS, GUILDFORD, GB, vol. 89, no. 10, 1 octobre 2010 (2010-10-01), pages 2873-2880, XP027136755, ISSN: 0016-2361 [extrait le 2010-03-17]
- SHIMIZU K I ET AL: "Enhanced production of hydroxymethylfurfural from fructose with solid acid catalysts by simple water removal methods", CATALYSIS COMMUNICATIONS, ELSEVIER SCIENCE, AMSTERDAM, NL, vol. 10, no. 14, 25 août 2009 (2009-08-25) , pages 1849-1853, XP026470071, ISSN: 1566-7367, DOI: 10.1016/J.CATCOM.2009.06.012 [extrait le 2009-06-18]
- CARNITI P ET AL: "Niobic acid and niobium phosphate as highly acidic viable catalysts in aqueous medium: Fructose dehydration reaction", CATALYSIS TODAY, ELSEVIER, NL, vol. 118, no. 3-4, 15 décembre 2006 (2006-12-15), pages 373-378, XP025116925, ISSN: 0920-5861, DOI: 10.1016/J.CATTOD.2006.07.024 [extrait le 2006-12-15]

## Description

La présente invention concerne la préparation de 5-hydroxyméthylfurfural (HMF) par déshydratation d'hexose, ou de ses dérivés ou de produits comprenant des hexoses ou dérivés d'hexose.

L'HMF et son principal dérivé, l'acide furane dicarboxylique (FDCA) sont des molécules présentant un énorme potentiel, notamment pour la production de polymères, notamment polyamides ou polyesters, du fait de leurs similitudes structurales avec l'acide téréphtalique, monomère habituellement utilisé.

L'HMF peut être obtenu par déshydratation en milieu aqueux de carbohydrates, notamment fructose, glucose ou matière cellulosique. Cependant, le rendement et la sélectivité sont faibles, notamment dans les milieux purement aqueux.
Le manque de sélectivité de la réaction de déshydratation des carbohydrates en HMF s'explique par la rapidité des réactions secondaires de polymérisation des intermédiaires réactionnels ou de l'HMF en milieu purement aqueux (par exemple formation d'humine). Différents procédés ont été développés pour tenter d'améliorer la conversion des carbohydrates et la sélectivité en HMF.
On connaît de FR2670209 la préparation d'HMF par déshydratation du fructose en milieu aqueux, en présence d'un catalyseur acide et d'un solvant d'extraction, permettant d'extraire l'HMF formé du milieu aqueux pour limiter sa dégradation. Le meilleur rendement obtenu est de 32% et la sélectivité en HMF est faible. L'acide lévulinique peut également être un sous produit important de la déshydratation du fructose formé par dégradation de l'HMF dans le cas d'un milieu réactionnel d'acidité trop élevée et, plus généralement, de conditions réactionnelles trop sévères.
On connait également de Antal et al (Carbohydrate Research, 1990, 199, 91-109) un procédé de préparation de HMF en milieu aqueux comprenant de faibles concentrations en acide organique (des quantités catalytiques inférieures à 5% en poids). Le rendement en HMF est cependant très faible.
Il est connu de GB 600 871 un procédé de préparation de HMF par réaction de carbohydrate avec une solution aqueuse comprenant un acide tel que l'acide chlorhydrique, l'acide phosphorique, l'acide fumarique et l'acide maléique.

US 2929 823 divulgue un procédé de préparation de HMF à partir de sucre, éventuellement en présence d'un catalyseur type acide lévulinique et éventuellement acide oxalique.
Selon EP 0230 250, il est connu de préparer du HMF par déshydratation du fructose en présence, par exemple, d'acide oxalique.
CN 101 381 351 divulgue un procédé de préparation de HMF par déshydratation de glucose en présence d'acide formique.
Haworth et al. (Journal of the Chemical Society, 1944, pages 667-670) divulgue un procédé de préparation de HMF à partir de fructose par hydrolyse à l'aide d'une solution aqueuse comprenant 0,25% d'acide oxalique.
L'utilisation de milieu biphasique, constitué d'une phase aqueuse réactive contenant de l'eau et du diméthylsulfoxyde (DMSO) et un catalyseur acide et une phase d'extraction (permettant d'extraire l'HMF formé du milieu aqueux pour limiter sa dégradation) à base de méthylisobutylcétone (MIBC ou MIBK) et de butanol ou de dichlorométhane, pour la préparation d'HMF à partir de fructose ou de glucose a également été mise en oeuvre (WO2007/146636 ; Dumeisc et al., Green Chem., 2007, 9, 342-350). Il a également été proposé l'utilisation de solvants à haut point d'ébullition comme la N-méthylpyrrolidone (NMP) et d'un catalyseur acide (US2006/0142599). Dans ces procédés, la conversion du fructose et du glucose et la sélectivité en HMF sont élevées. Cependant, ces procédés nécessitent l'emploi de solvants pouvant être toxiques, nécessitant donc des purifications de l'HMF obtenu, et dont les températures d'ébullition sont élevées, ce qui fait de ces procédés des procédés complexes et coûteux.
On connaît également l'emploi d'un acide faible, tel que l'acide borique, associé à l'ajout de sel et à l'utilisation d'un solvant d'extraction tel que la méthylisobutylcétone. Les rendements en HMF à partir du fructose sont de 60% (Thomas S.Hansen et al., Green Chem., 2010, 13(1), 109-114).
La mise en oeuvre de liquide ionique a également été proposée avec un catalyseur à base de lanthanide (Stählberg et al., Green Chem, 2010, 12(2), 321-325).
Enfin, on connaît de WO2009/076627, la préparation d'HMF par hydrolyse de son ester obtenu à partir du fructose dans une colonne comprenant une résine de type Amberlyst et en présence d'acide acétique. Cependant, il n'est pas décrit un procédé permettant d'obtenir directement l'HMF à partir du fructose.
Ainsi, afin d'augmenter la conversion en carbohydrate et la sélectivité en HMF des techniques de plus en plus complexes ont été développées.

La production d'HMF d'une façon sélective est complexe et sa purification est difficile en raison de l'instabilité de cette molécule. Il est également difficile d'obtenir l'HMF à bas coût. Pour ces différentes raisons il n'y a toujours pas de production d'HMF à l'échelle industrielle.

Il y a donc un intérêt à fournir un procédé de préparation d'HMF qui réponde aux inconvénients des procédés de l'état de la technique.
Un objectif de la présente invention est de fournir un procédé de préparation d'HMF avantageux d'un point de vue industriel.
Un autre objectif de la présente invention est de fournir un procédé de préparation d'HMF avec une sélectivité et une conversion élevée.
Un autre objectif encore de la présente invention est de fournir un procédé de préparation d'HMF permettant de réduire, voire de supprimer, la formation de produits secondaires notamment de type humine.
Un objectif de l'invention est également de fournir un procédé mettant en oeuvre des solvants non toxiques et pouvant facilement être éliminés et/ou recyclés, avec ou sans catalyseurs.
D'autres objectifs apparaîtront à la lecture de la description de l'invention qui suit.
Tous ces objectifs sont remplis par l'invention qui concerne un procédé de préparation du 5-hydroxyméthylfurfural (HMF) par réaction d'hexose dans l'eau et en présence d'acide carboxylique, l'acide carboxylique étant présent en une quantité de 10 à 50 % en poids par rapport au poids total eau + acide carboxylique, et étant choisi parmi :
- les acides de formule RCOOH dans laquelle R représente un atome d'hydrogène ou une chaîne alkyle, linéaire ou ramifiée, en C₁ à C₅, éventuellement substituée par un ou plusieurs groupes OH ;
- leurs mélanges.

Selon l'invention, on entend par « hexose », outre des composés de formule chimique C₆H₁₂O₆ cycliques tels que le glucose ou le fructose ou leurs mélanges, des dérivés d'hexoses et des produits comprenant des hexoses ou leurs dérivés. Les compositions comprenant des hexoses sont également visées par le procédé de l'invention.
On entend par « dérivés d'hexose », des composés comprenant dans leur structure au moins un motif d'hexose, ils peuvent notamment être obtenus par polymérisation d'hexoses. Les dérivés d'hexose selon l'invention sont notamment les polymères du glucose, notamment l'amidon, la cellulose ou contenant au moins un motif hexose, l'hémicellulose.

Selon l'invention, les produits comprenant des hexoses ou leurs dérivés peuvent être représentés par la biomasse lignocellulosique qui correspond à un assemblage de cellulose, d'hémicellulose et de lignine ; ou le papier, qui comprend de la cellulose, notamment le papier recyclé.
Les celluloses, hémicelluloses, biomasse lignocellulosique et papier peuvent être utilisés tels quels dans le procédé de l'invention. Ils peuvent être également pré-traités, notamment par hydrolyse plus ou moins poussée, avant d'être soumis à l'étape en présence d'acide carboxylique de l'invention.
De façon préférée, l'hexose est le fructose.

Les acides carboxyliques sont des monoacides. Ils sont notamment choisis parmi :
- les acides de formule R-COOH dans laquelle R représente un atome d'hydrogène ou une chaîne alkyle, linéaire ou ramifiée, en C₁ à C₅, de préférence en C₁ à C₃, éventuellement substituée par un ou plusieurs groupes OH ; ou
- leurs mélanges.

De préférence, l'acide carboxylique est l'acide formique, l'acide acétique, l'acide propionique, l'acide lactique ou leurs mélanges. De manière plus préférée, l'acide carboxylique est l'acide formique, l'acide acétique, l'acide lactique ou leurs mélanges.

De façon plus préférée, l'acide carboxylique est l'acide formique, l'acide acétique ou leur mélange. Ces deux acides présentent l'avantage d'être stables dans le milieu réactionnel et d'être facilement éliminés notamment par évaporation sous vide.
De façon avantageuse, lorsque l'acide carboxylique est l'acide formique, la conversion de l'hexose, e.g. fructose, de départ peut être totale. Il sera ainsi possible d'obtenir une composition d'acide formique riche en HMF qui pourra être directement utilisée, notamment pour la préparation de polymère, par exemple de polyamides ou de polyesters.
De façon avantageuse, lorsque l'acide carboxylique est l'acide acétique, la conversion de l'hexose, e.g. fructose, de départ et la purification de l'HMF obtenu est facilitée du fait de la volatilité de l'acide acétique. Il sera ainsi possible d'obtenir une composition d'acide acétique riche en HMF qui pourra être directement utilisée, notamment pour la préparation de polymère, par exemple de polyamides ou de polyesters ; ou de purifier cette solution pour obtenir l'HMF.

La présence de l'acide selon l'invention, en comparaison avec le même procédé mis en oeuvre uniquement en présence d'eau, permet d'augmenter significativement la conversion de l'hexose, e.g. fructose, et la sélectivité en HMF.
L'homme du métier, selon qu'il préfère favoriser la conversion de l'hexose, e.g. fructose, ou la sélectivité en HMF ou avoir un bon compromis entre ces deux caractéristiques pourra déterminer la proportion et la nature de l'acide à intégrer au milieu réactionnel. La quantité d'acide ne doit pas être trop importante au risque d'augmenter la production de produits secondaires, notamment d'humine.
Les inventeurs ont mis en évidence que le contrôle de la concentration en acide permet de manière particulièrement avantageuse et surprenante d'obtenir un rendement et une sélectivité en HMF importants. A faible concentration en acide, le rendement en HMF est très faible voire nul et à forte concentration en acide le rendement en HMF est faible et l'acide lévullinique est obtenu en tant que sous produit en quantités importantes.
Ainsi, la quantité d'acide est comprise entre 10 et 50 % en poids, par exemple 20 % ou 10 % en poids par rapport au poids total eau + acide carboxylique.

Il a de plus été montré par les inventeurs que, de façon surprenante, les acides carboxyliques, et en particulier les acides formique et acétique, notamment l'acide acétique, permettaient de stabiliser l'HMF formé dans le milieu aqueux.
Ainsi l'HMF obtenu dans le milieu aqueux d'acide acétique après 3h à 150°C ne se dégrade qu'à hauteur de 25 % alors que dans un milieu acidifié au même pH (1,71) par ajout d'HCI, 60 % de l'HMF est dégradé.

Dans le procédé selon l'invention, l'eau est présente de préférence en une quantité entre 50 et 90 % en poids, par exemple 80 ou 90 % en poids par rapport au poids total eau + acide carboxylique.

Dans le procédé de l'invention, la quantité d'hexose, e.g. fructose, dépend de sa solubilité dans le mélange d'eau et d'acide carboxylique. Elle est généralement comprise entre 0,5 et 35 %, de préférence entre 1 % et 30 %, notamment entre 0,5 et 25%, de préférence entre 1 et 15%, par exemple 1 %, par rapport au poids total eau + acide carboxylique.

Les inventeurs ont également montré que le pH pouvait avoir une influence sur la réaction. Ainsi, si le pH est trop acide, une quantité importante de sous-produits, notamment d'humine, peut se former.

De manière avantageuse, le pH du milieu réactionnel en début de réaction est compris entre 1 et 3, de préférence entre 1,5 et 2,5. Il est par exemple de 1,7.

Le procédé selon l'invention peut être mené à une température comprise entre 100 et 200°C, de préférence entre 120 et 180°C, par exemple entre 150 et 180°C.

Le procédé selon l'invention peut être mis en oeuvre à pression atmosphérique ou sous pression d'un gaz inerte, par exemple l'hélium, jusqu'à une pression de 3,5 MPa (soit 35 bars).

Le procédé de l'invention peut avantageusement être mis en oeuvre en présence d'un catalyseur acide hétérogène. De préférence, le catalyseur est choisi parmi l'acide tungstophosphorique, de préférence dispersé sur hydroxyde de niobium (NbOH); l'hydroxyde de niobium ; les zircones sulfatées ; les sels acides de césium de l'acide tungstophosphorique (Cs₂HPW₁₂O₄₀); le dioxyde de titane ; les charbons sulfonés ; les charbons fonctionnalisés, par exemple par des groupements carboxyliques, par exemple suite à une oxydation, par exemple oxydation par de l'eau de javel ; ou leurs mélanges. L'ajout de ces catalyseurs permet notamment de manière avantageuse d'augmenter la conversion en hexose, e.g. fructose, et la sélectivité en HMF.
De façon particulièrement préférée le catalyseur est un charbon sulfoné ou un charbon fonctionnalisé.
Lorsqu'il est présent, la quantité de catalyseur est de préférence comprise entre 2 et 100% en poids, de préférence entre 2 et 10 % en poids, par exemple 5 % en poids par rapport au poids d'hexose, e.g. fructose.

De manière avantageuse, le procédé de l'invention ne nécessite pas la mise en oeuvre de solvant autre que l'eau. Ainsi et de préférence, le procédé de l'invention est mis en oeuvre en l'absence de solvant organique.

Le procédé selon l'invention peut être mis en oeuvre en batch ou en continu. Il sera avantageusement mis en oeuvre en continu. Si un catalyseur acide hétérogène est utilisé, le procédé sera avantageusement mis en oeuvre en continu sur un lit fixe de catalyseur.

De façon avantageuse, le procédé de l'invention permet d'obtenir des conversions en hexose, notamment fructose, supérieures à 50 %, de préférence supérieures à 90 %.

De façon avantageuse, le procédé de l'invention permet d'obtenir une sélectivité en HMF supérieure à 50 %, de préférence supérieure à 75 %.

Il est également décrit des solutions aqueuses d'acide carboxylique comprenant du HMF, de préférence comprenant de 0,1 à 10 % en poids d'HMF, par exemple de 0,3 à 6 % en poids d'HMF et pouvant comprendre de 0 à 5 % en poids d'hexose, e.g. fructose, par exemple de 0 à 0,5 % en poids d'hexose, e.g. fructose. Ces solutions sont susceptibles d'être obtenues par et grâce au procédé de l'invention.
Il est également décrit une solution aqueuse d'acide formique ou d'acide acétique comprenant du HMF, de préférence comprenant de 0,1 à 15 %, de préférence e 0,1 à 10% en poids d'HMF et pouvant comprendre de 0 à 5 % en poids d'hexose, e.g. fructose. De préférence, cette solution ne comprend pas d'hexose, e.g. fructose, et peut être utilisée directement, notamment pour la préparation de polymères tels que les polyamides et les polyesters.
La figure 1 représente l'influence de la nature de l'acide carboxylique ajouté au milieu aqueux sur la conversion du fructose à 150°C (Ac = acide ; AcAc = acide acétique).
La figure 2 représente l'influence de la nature du catalyseur hétérogène ajouté à la solution aqueuse d'acide acétique sur la conversion du fructose et le rendement en HMF à 150°C.
La figure 3 représente l'influence de la teneur en acide acétique dans le milieu réactionnel aqueux sur la conversion du fructose en HMF en absence d'ajout de catalyseur hétérogène à 150°C (Rendt = rendement ; cata=catalyseur)
La figure 4 représente l'influence de la teneur en acide formique dans le milieu réactionnel aqueux sur la conversion du fructose en HMF en absence d'ajout de catalyseur hétérogène à 150°C.
La figure 5 représente l'influence de la teneur en acide acétique dans le milieu réactionnel aqueux sur la conversion du fructose en HMF en présence de 5 % d'hydroxyde de niobium à 150°C.
La figure 6 représente l'influence du pH sur la conversion du fructose en HMF à 150°C, ajusté finement dans le domaine des pH acides, par ajout d'HCI au milieu aqueux.
La figure 7 représente la conversion de l'HMF dans les milieux : eau pure (pH=6,7) ; 20 % acide acétique dans l'eau ; eau acidifiée par de l'HCl à un pH équivalent au milieu 20 % acide acétique dans l'eau (pH=1,71).
La figure 8 représente l'influence de la teneur en fructose dans le milieu réactionnel aqueux contenant 20 % d'acide acétique sur la conversion du fructose en HMF à 150°C.
La figure 9 représente l'influence de la température sur la conversion du fructose et le rendement en HMF dans le milieu réactionnel eau-acide acétique.
La figure 10 représente l'influence de la température sur la conversion du fructose et le rendement en HMF dans le milieu réactionnel eau-acide formique.

La présente invention va maintenant être décrite à l'aide d'exemples non limitatifs.

### Exemple 1 : Influence de l'acide carboxylique

La synthèse de l'HMF est réalisée dans un autoclave de 100ml. Les quantités suivantes sont introduites dans le réacteur : 60 g de solution aqueuse, 0,6 g de fructose.
Les solutions aqueuses utilisées sont pures ou comprennent des acides carboxyliques. Les solutions aqueuses d'acide carboxylique ont les compositions suivantes :
- 12g d'acide lactique ajoutés à 48 g d'eau distillée ; ou
- 12g d'acide acétique ajoutés à 48 g d'eau distillée ; ou
- 12g d'acide formique ajoutés à 48 g d'eau distillée.

2 MPa (20 bars) d'hélium sont introduits dans l'autoclave. Le milieu réactionnel est agité au moyen d'un agitateur magnétique. Le milieu réactionnel est porté à la température de réaction au moyen de résistances électriques régulées à 150°C. Après 2h de réaction à 150°C l'autoclave est refroidi au moyen d'un bain de glace. La conversion du fructose et le rendement molaire en HMF sont déterminés par analyse HPLC-RID (colonne : COREGEL 87C).

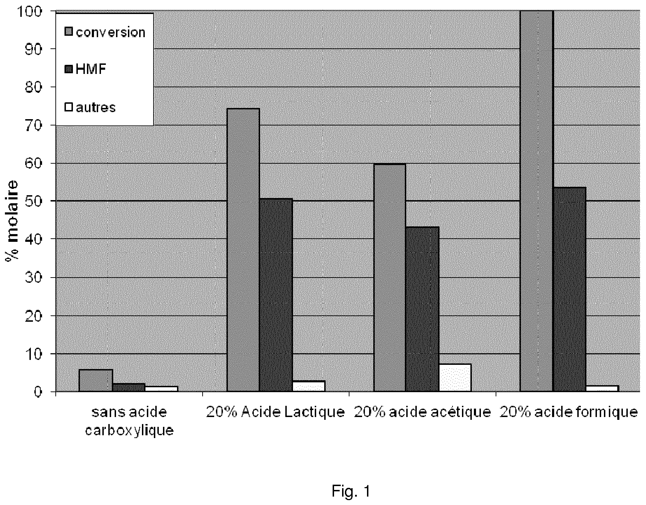

Les résultats obtenus sont présentés à la figure 1. Les résultats montrent que l'ajout d'acide carboxylique permet d'augmenter la conversion du fructose et la sélectivité en HMF. Les résultats montrent également que la conversion du fructose est totale avec l'acide formique et que le meilleur compromis entre conversion et sélectivité en HMF est obtenu avec l'acide acétique.

### Exemple 2 : Influence de l'ajout de catalyseurs acides hétérogènes

La synthèse de l'HMF est réalisée dans un autoclave de 100ml. Les quantités suivantes sont introduites dans le réacteur : 48 g d'eau distillée, 12 g d'acide acétique, 0,6 g de fructose, 30 mg de catalyseur. 2 MPa (20 bars) d'hélium sont introduits dans le milieu réactionnel. Le milieu réactionnel est agité au moyen d'un agitateur magnétique. Le milieu réactionnel est porté à la température de réaction au moyen de résistances électriques régulées à 150°C. Après 2h de réaction à 150°C l'autoclave est refroidi au moyen d'un bain de glace. Le catalyseur est séparé par filtration. La conversion du fructose et le rendement molaire en HMF sont déterminés par analyse HPLC-RID (colonne : COREGEL 87C).

Le procédé a été mis en oeuvre en l'absence de catalyseur et en présence de différents catalyseurs, à savoir la zircone tungstée (ZrW), les sels acides de césium de l'acide tungstophosphorique (Cs₂H : Cs₂HPW₁₂O₄₀), le dioxyde de titane (TiO₂), le charbon sulfoné (C/Sulfoné), l'hydroxyde de niobium (NbOH) et le charbon fonctionnalisé par des groupes carboxyliques suite à une oxydation par l'eau de javel.

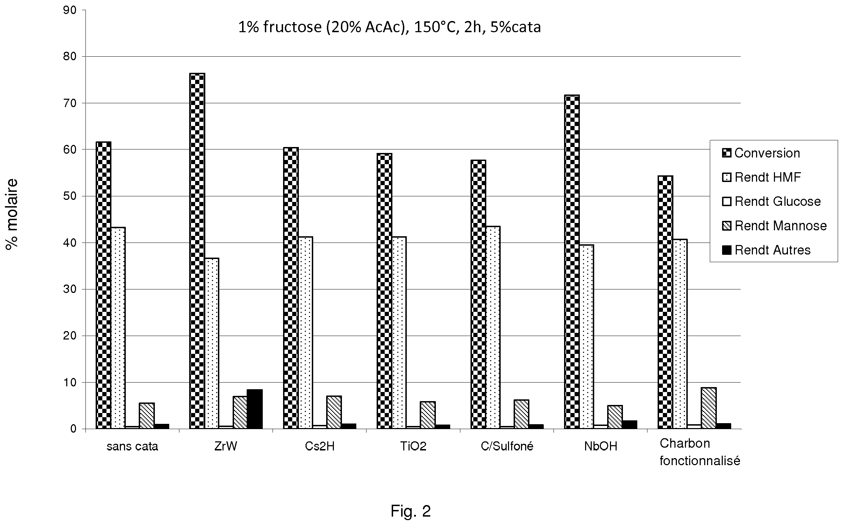

Les résultats obtenus sont présentés à la figure 2. Les résultats montrent que l'ajout de catalyseur permet d'augmenter la conversion du fructose. Les résultats montrent notamment que le meilleur compromis entre conversion et sélectivité en HMF est obtenu avec le charbon sulfoné ou le charbon fonctionnalisé.

### Exemple 3 : Influence de la teneur en acide carboxylique

La synthèse de l'HMF est réalisée dans un autoclave de 100ml. Les quantités suivantes sont introduites dans le réacteur : 60 g d'eau distillée ou 60 g d'une solution aqueuse pouvant contenir 5%, 10 %, 20 %, 30%, 50 % en poids d'acide carboxylique ou 60 g d'acide carboxylique pur et 0,6 g de fructose. 2 MPa (20 bars) d'hélium sont introduits dans l'autoclave. Le milieu réactionnel est agité au moyen d'un agitateur magnétique. Le milieu réactionnel est porté à la température de réaction au moyen de résistances électriques régulées à 150°C. Après 2h de réaction à 150°C, l'autoclave est refroidi au moyen d'un bain de glace. La conversion du fructose et le rendement molaire en HMF sont déterminés par analyse HPLC-RID (colonne : COREGEL 87C).

Le procédé a été mis en oeuvre avec de l'acide carboxylique en différentes proportions à savoir 5%,10 %, 20 %, 30% et 50 % en poids du milieu réactionnel aqueux comprenant le fructose et sans acide carboxylique. Le procédé a également été mis en oeuvre avec un milieu réactionnel comprenant uniquement l'acide carboxylique et le fructose.

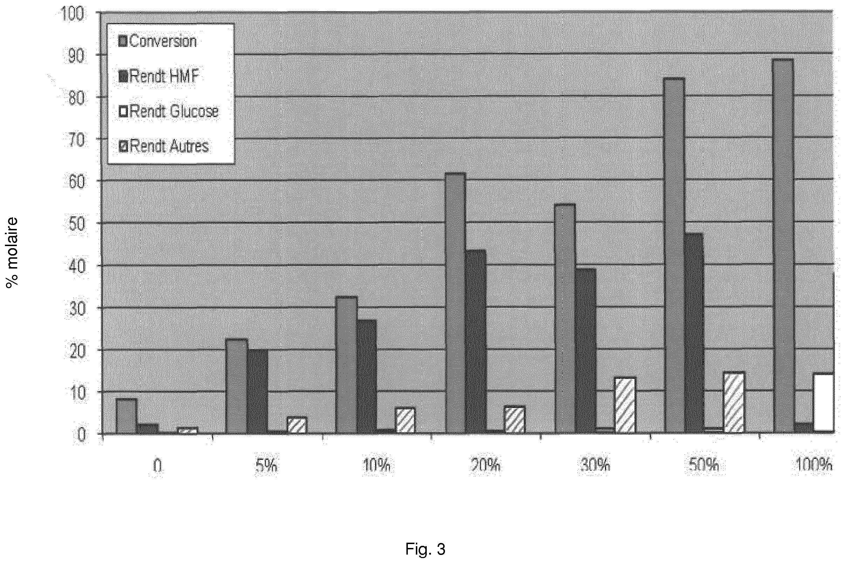

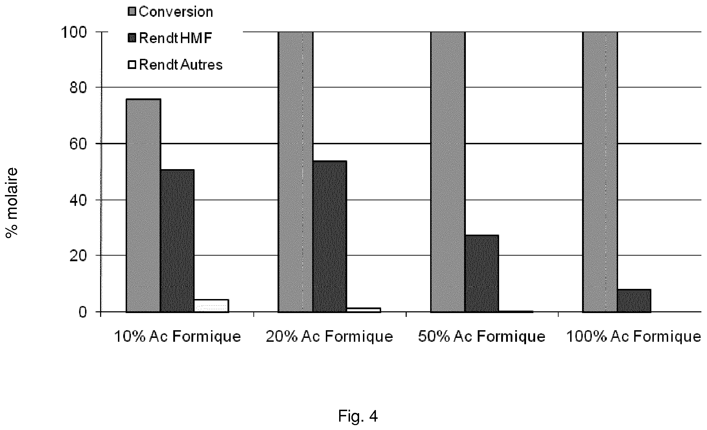

Les résultats obtenus sont présentés à la figure 3 et à la figure 4 pour montrer respectivement l'influence de la teneur en acides acétique et formique. Les résultats montrent que pour des quantités élevées d'acide carboxylique, la conversion en fructose augmente, mais la sélectivité en HMF décroît. Ainsi, lorsque la réaction est mise en oeuvre avec 100 % d'acide carboxylique, une quantité très faible d'HMF est obtenue.
Le meilleur compromis entre conversion du fructose et sélectivité en HMF est obtenu pour 20 % d'acide carboxylique pour l'acide acétique et 10% pour l'acide formique.

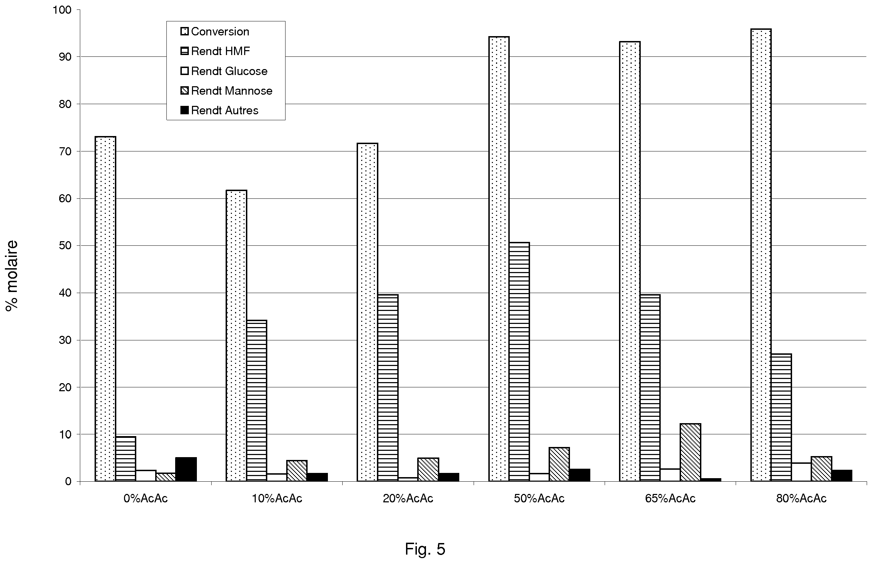

Le même procédé a été mis en oeuvre avec 5 % d'hydroxyde de niobium par rapport à la quantité de fructose. Les résultats obtenus sont présentés à la figure 5. Les résultats montrent que pour des quantités élevées d'acide carboxylique, la conversion en fructose augmente, mais la sélectivité en HMF décroît. Ainsi, lorsque la réaction est mise en oeuvre avec plus de 80 % d'acide carboxylique, une quantité faible d'HMF est obtenue. Le meilleur compromis entre conversion du fructose et sélectivité en HMF est obtenu pour 20 % d'acide acétique.

### Exemple 4 : Influence du pH du milieu réactionnel de départ

La synthèse de l'HMF est réalisée dans un autoclave de 100ml. Les quantités suivantes sont introduites dans le réacteur : 60 g d'eau distillée auxquels sont ajoutés de l'HCl pour finement ajuster le pH entre 3 et 1 et 0,6 g de fructose. 2 MPa (20 bars) d'hélium sont introduits dans l'autoclave. Le milieu réactionnel est agité au moyen d'un agitateur magnétique. Le milieu réactionnel est porté à la température de réaction au moyen de résistances électriques régulées à 150°C. Après 2h de réaction à 150°C, l'autoclave est refroidi au moyen d'un bain de glace. La conversion du fructose et le rendement molaire en HMF sont déterminés par analyse HPLC-RID (colonne : COREGEL 87C).

L'influence du pH en début de réaction a été étudiée. Pour cela, le pH du milieu réactionnel de départ a été ajusté par ajout d'HCl.

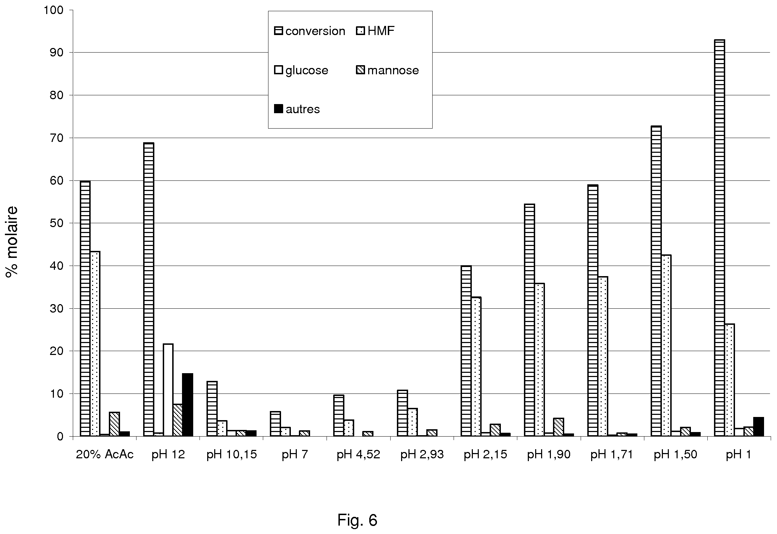

Les résultats sont présentés à la figure 6. Les résultats montrent qu'à pH faible la conversion en fructose augmente, mais la sélectivité en HMF diminue. Les résultats montrent qu'un bon compris entre conversion en fructose et sélectivité en HMF est obtenu dans une plage de pH comprise entre 1,5 et 2,15, notamment à un pH de 1,71 correspondant à un milieu comprenant 20 % d'acide acétique.

### Exemple 5 : Stabilité de l'HMF

L'étude de la stabilité de l'HMF à 150°C en fonction de la composition du milieu aqueux est étudiée dans un autoclave de 100ml. Les quantités suivantes sont introduites dans le réacteur : 60 g d'eau distillée ou 60 g d'eau distillée ajustée au pH 1,71 par ajout d'HCI ou 48 g d'eau distillée additionnées de 12 g d'acide acétique. A ces solutions aqueuses est ajouté 0,6 g de fructose. 2 MPa (20 bars) d'hélium sont introduits dans l'autoclave. Le milieu réactionnel est agité au moyen d'un agitateur magnétique. Le milieu réactionnel est porté à la température de réaction au moyen de résistances électriques régulées à 150°C. Des prélèvements du milieu réaction sont effectués en début de réaction, puis chaque heure, pour analyse. La conversion du fructose et le rendement molaire en HMF sont déterminés par analyse HPLC-RID (colonne : COREGEL 87C).

La stabilité de l'HMF a été étudiée dans l'eau, dans un milieu aqueux comprenant 20 % d'acide acétique et dans une eau acidifiée par de l'HCl à un pH équivalent à celui de la solution aqueuse d'acide acétique 20 % (c'est à dire à un pH de 1,71).

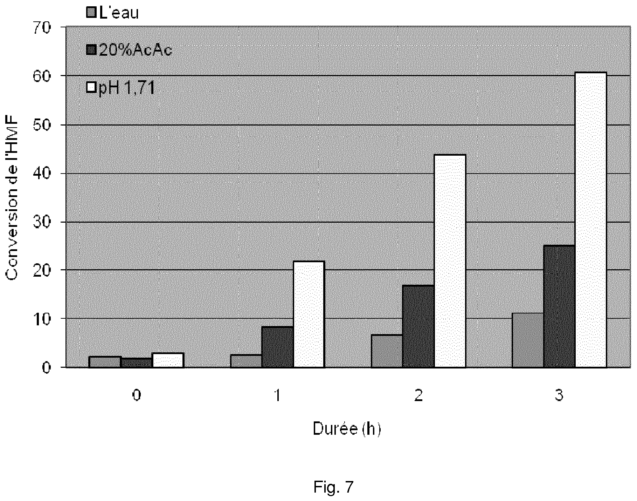

Les résultats sont présentés à la figure 7 et montrent le pourcentage d'HMF converti en fonction du temps.
Les résultats montrent une stabilisation de l'HMF dans le milieu aqueux d'acide acétique par rapport au milieu aqueux acidifié par l'acide minéral HCl. Les résultats montrent ainsi que l'utilisation d'acide carboxylique pour la préparation d'HMF permet d'obtenir une conversion des hexoses et un rendement en fructose élevés tout en stabilisant l'HMF obtenu.

### Exemple 6 : Influence de la teneur en fructose dans le milieu réactionnel de départ.

La synthèse de l'HMF est réalisée dans un autoclave de 100ml. Les quantités suivantes sont introduites dans le réacteur : 48 g d'eau distillée et 12 g d'acide acétique auxquels sont ajoutés 0,6 g de fructose (1 %) ou 3 g de fructose (5 %) ou 6 g de fructose (10 %) ou 9 g de fructose (15 %) ou 18 g de fructose (30%) . La réaction a également été menée avec 48 g d'eau distillée et 0,48 g d'acide acétique (1%) auxquels est ajouté 18 g de fructose (30%). 2 MPa (20 bars) d'hélium sont introduits dans l'autoclave. Le milieu réactionnel est agité au moyen d'un agitateur magnétique. Le milieu réactionnel est porté à la température de réaction au moyen de résistances électriques régulées à 150°C. Après 2h de réaction à 150°C, l'autoclave est refroidi au moyen d'un bain de glace. La conversion du fructose et le rendement molaire en HMF sont déterminés par analyse HPLC-RID (colonne : COREGEL 87C).

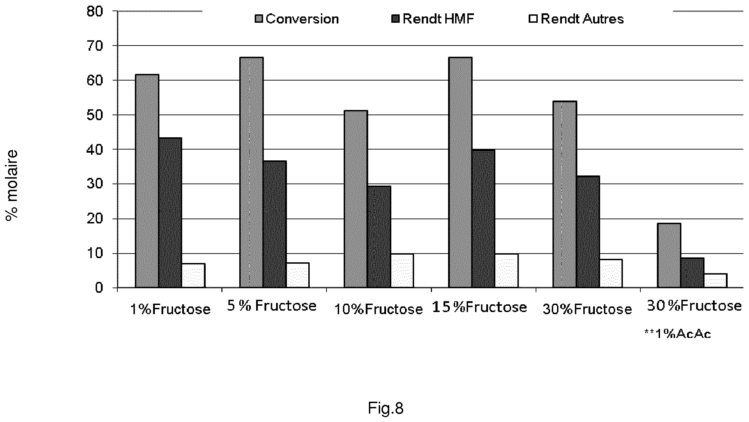

Les résultats sont présentés sur la figure 8. Les résultats montrent que la conversion du fructose et le rendement en HMF dépendent peu de la concentration en fructose. Les résultats montrent que le rendement en HMF obtenu à partir d'une solution concentrée en fructose, notamment 15 %, atteint 40%, valeur proche du rendement obtenu à partir d'une solution à 1 % en fructose. La concentration en fructose est limitée par la solubilité du fructose dans la solution aqueuse d'acide carboxylique.

### Exemple 7: Influence de la température sur la production d'HMF en milieu eau-acide acétique

La synthèse de l'HMF est réalisée dans un autoclave de 100ml. Les quantités suivantes sont introduites dans le réacteur : 48 g d'eau distillée auxquels sont ajoutés 12 g d'acide acétique et 0,6 g de fructose (1 %). 2 MPa (20 bars) d'hélium sont introduits dans l'autoclave. Le milieu réactionnel est agité au moyen d'un agitateur magnétique. Le milieu réactionnel est porté à la température de réaction au moyen de résistances électriques régulées. Les températures suivantes sont étudiées : 100°C, 120°C, 150°C, 180°C. Après 2h de réaction, l'autoclave est refroidi au moyen d'un bain de glace. La conversion du fructose et le rendement en HMF sont déterminés par analyse HPLC-RID (colonne : COREGEL 87C).

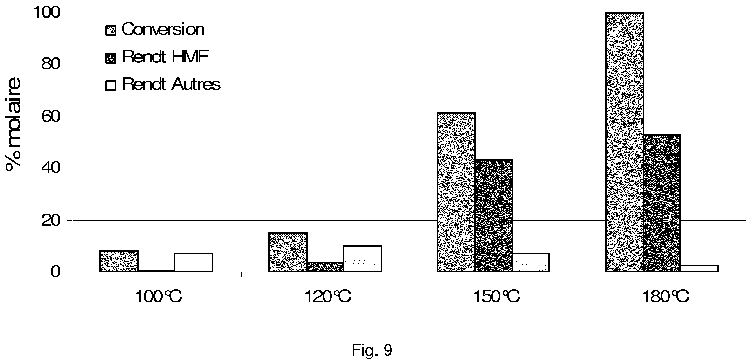

Les résultats sont présentés sur la figure 9. Les résultats montrent que la conversion du fructose et le rendement en HMF sont faibles aux températures de réaction de 100°C et 120°C. La figure 9 montre une augmentation de la conversion en fructose et du rendement en HMF avec l'augmentation de température. Le meilleur compromis entre la conversion en fructose et le rendement en HMF est obtenu à la température de 150°C. On observe aussi qu'à la température de 180°C, la conversion du fructose est totale et le rendement molaire en HMF atteint 54 %.

### Exemple 8 : Influence de la température sur la production d'HMF en milieu eau-acide formique

La synthèse de l'HMF est réalisée dans un autoclave de 100ml. Les quantités suivantes sont introduites dans le réacteur : 48 g d'eau distillée auxquels sont ajoutés 12 g d'acide formique et 0,6 g de fructose (1 %). 2 MPa (20 bars) d'hélium sont introduits dans l'autoclave. Le milieu réactionnel est agité au moyen d'un agitateur magnétique. Le milieu réactionnel est porté à la température de réaction au moyen de résistances électriques régulées. Les températures suivantes sont étudiées : 100°C, 120°C, 150°C, 180°C. Après 2h de réaction, l'autoclave est refroidi au moyen d'un bain de glace. La conversion du fructose et le rendement molaire en HMF sont déterminés par analyse HPLC-RID (colonne: COREGEL 87C).

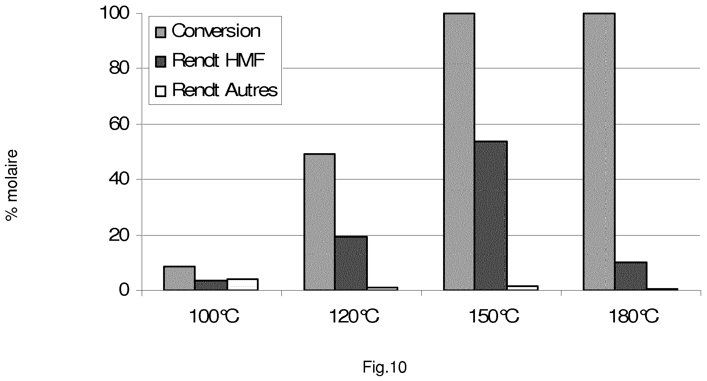

Les résultats sont présentés sur la figure 10. Les résultats montrent que la conversion du fructose et le rendement en HMF sont faibles à la température de réaction de 100°C. La figure 10 montre une augmentation de la conversion en fructose et du rendement en HMF avec l'augmentation de température jusqu'à 150°C, température à laquelle la conversion est totale. A la température de réaction de 180°C, la conversion du fructose est totale mais le rendement molaire en HMF est faible, 10 %. Le rendement le plus élevé en HMF, 54 %, est obtenu à conversion totale du fructose, à la température de réaction de 150°C.

## Revendications

1. Procédé de préparation de 5-hydroxyméthylfurfural (HMF) par réaction d'hexose dans l'eau et en présence d'acide carboxylique, l'acide carboxylique étant présent en une quantité de 10 à 50 % en poids par rapport au poids total eau + acide carboxylique, l'acide carboxylique étant choisi parmi :
- les acides de formule R-COOH dans laquelle R représente un atome d'hydrogène ou une chaîne alkyle, linéaire ou ramifiée, en C₁ à C₅, éventuellement substituée par un ou plusieurs groupes OH ;
- leurs mélanges.

2. Procédé selon la revendication 1, pour lequel l'hexose est le fructose, le glucose ou les dérivés des hexoses choisis parmi la cellulose, l'hémicellulose, la lignine et le papier.

3. Procédé selon la revendication 1 ou 2, pour lequel l'hexose est le fructose.

4. Procédé selon l'une quelconque des revendications 1 à 3, pour lequel l'acide est l'acide formique, l'acide acétique, l'acide lactique, l'acide propionique ou leurs mélanges.

5. Procédé selon l'une quelconque des revendications 1 à 4, pour lequel l'acide est l'acide formique, l'acide acétique ou leur mélange.

6. Procédé selon l'une quelconque des revendications 1 à 5, pour lequel l'acide carboxylique est présent en une quantité de 20 % en poids par rapport au poids total eau + acide carboxylique.

7. Procédé selon l'une quelconque des revendications 1 à 6, pour lequel l'eau est présente en une quantité de 50 à 90% en poids par rapport au poids total eau + acide carboxylique.

8. Procédé selon l'une quelconque des revendications 1 à 7, lequel est mis en oeuvre en présence d'un catalyseur acide hétérogène.

9. Procédé selon la revendication 8, pour lequel le catalyseur acide est choisi parmi l'acide tungstophosphorique ; l'hydroxyde de niobium ; les zircones sulfatées ; les sels acides de césium de l'acide tungstophosphorique ; le dioxyde de titane ; les charbons sulfonés ; les charbons fonctionnalisés; ou leurs mélanges.

10. Procédé selon la revendication 8 ou 9, pour lequel la quantité de catalyseur est comprise entre 2 et 100 % en poids par rapport au poids d'hexose.

11. Procédé selon l'une quelconque des revendications 1 à 10, pour lequel la quantité d'hexose est comprise entre 0.5 % et 35 % en poids par rapport au poids total eau + acide carboxylique.

12. Procédé selon l'une quelconque des revendications 1 à 9, pour lequel le pH en début de réaction est compris entre 1 et 3.

13. Procédé selon l'une quelconque des revendications 1 à 10, mené à une température comprise entre 100 et 200°C.

14. Procédé selon l'une quelconque des revendications 1 à 13, mené en l'absence de solvant organique.

15. Procédé selon l'une quelconque des revendications 1 à 14, mené en continu.

## Patentansprüche

1. Verfahren zur Herstellung von 5-Hydroxymethylfurfural (HMF) durch Reaktion von Hexose in Wasser und in Anwesenheit von Carbonsäure, wobei die Carbonsäure in einer Menge von 10 bis 50 Gew% mit Bezug auf das Gesamtgewicht von Wasser + Carbonsäure vorhanden ist, wobei die Carbonsäure ausgewählt ist aus:
- den Säuren der Formel R-COOH, wobei R ein Wasserstoffatom oder eine Alkylkette darstellt, linear oder verzweigt, mit C₁ bis C₅, eventuell substituiert durch eine oder mehrere OH-Gruppen;
- ihren Mischungen.

2. Verfahren nach Anspruch 1, wobei die Hexose Fructose, Glucose oder die Derivate der Hexosen ist, ausgewählt aus Cellulose, Hemicellulose, Lignin und Papier.

3. Verfahren nach Anspruch 1 oder 2, wobei die Hexose Fructose ist.

4. Verfahren nach einem beliebigen der Ansprüche 1 bis 3, wobei die Säure Ameisensäure, Essigsäure, Milchsäure, Propionsäure und ihre Mischungen ist.

5. Verfahren nach einem beliebigen der Ansprüche 1 bis 4, wobei die Säure Ameisensäure, Essigsäure oder ihre Mischung ist.

6. Verfahren nach einem beliebigen der Ansprüche 1 bis 5, wobei die Carbonsäure in einer Menge von 20 Gew% mit Bezug auf das Gesamtgewicht von Wasser + Carbonsäure vorhanden ist.

7. Verfahren nach einem beliebigen der Ansprüche 1 bis 6, wobei Wasser in einer Menge von 50 bis 90 Gew% mit Bezug auf das Gesamtgewicht von Wasser + Carbonsäure vorhanden ist.

8. Verfahren nach einem beliebigen der Ansprüche 1 bis 7, das in Anwesenheit eines heterogenen Säurekatalysators durchgeführt wird.

9. Verfahren nach Anspruch 8, wobei der Säurekatalysator ausgewählt ist aus Wolframphosphorsäure, Niobhydroxid, sulfatierten Zirkonen, sauren Cäsiumsalzen der Wolframphosphorsäure; Titandioxid; sulfonierten Kohlen; funktionalisierten Kohlen; oder ihren Mischungen.

10. Verfahren nach Anspruch 8 oder 9 wobei die Menge an Katalysator im Bereich zwischen 2 und 100 Gew% mit Bezug auf das Gewicht von Hexose liegt.

11. Verfahren nach einem beliebigen der Ansprüche 1 bis 10, wobei die Menge Hexose im Bereich zwischen 0,5 und 35 Gew% mit Bezug auf das Gesamtgewicht von Wasser + Carbonsäure liegt.

12. Verfahren nach einem beliebigen der Ansprüche 1 bis 9, wobei der pH-Wert am Beginn der Reaktion im Bereich zwischen 1 und 3 liegt.

13. Verfahren nach einem beliebigen der Ansprüche 1 bis 10, das bei einer Temperatur im Bereich zwischen 100 und 200 °C durchgeführt wird.

14. Verfahren nach einem beliebigen der Ansprüche 1 bis 13, das in Abwesenheit von organischem Lösemittels durchgeführt wird.

15. Verfahren nach einem beliebigen der Ansprüche 1 bis 14, das ununterbrochen durchgeführt wird.

## Claims

1. Process for preparing 5-hydroxymethylfurfural (HMF) by reacting hexose in water and in the presence of carboxylic acid, the carboxylic acid being present in a quantity of 10 to 50% by weight with respect to the total water + carboxylic acid weight, the carboxylic acid being selected from the group consisting of.
- acids of formula R-COOH wherein R represents a hydrogen atom or a C₁ to C₅ alkyl chain, linear or branched, optionally substituted by one or more OH groups;
- mixtures thereof.

2. Process according to claim 1, for which the hexose is fructose, glucose, or hexose derivatives selected from the group consisting of cellulose, hemicellulose, lignin and paper.

3. Process according to claim 1 or 2, for which the hexose is fructose.

4. Process according to any one of claims 1 to 3, for which the acid is formic acid, acetic acid, lactic acid, propionic acid, or mixtures thereof.

5. Process according to any one of claims 1 to 4, for which the acid is formic acid, acetic acid or a mixture thereof.

6. Process according to any one of claims 1 to 5, for which the carboxylic acid is present in a quantity of 20% by weight with respect to the total water + carboxylic acid weight.

7. Process according to any one of claims 1 to 6, for which the water is present in a quantity of 50 to 90% by weight with respect to the total water + carboxylic acid weight.

8. Process according to any one of claims 1 to 7, which is implemented in the presence of a heterogeneous acid catalyst.

9. Process according to claim 8, for which the acid catalyst is chosen from tungstophosphoric acid; niobium hydroxide; sulfated zirconia; acidic cesium salts of tungstophosphoric acid; titanium dioxide; sulfonated carbon; functionalized carbon; or mixtures thereof.

10. Process according to claim 8 or 9, for which the quantity of catalyst is between 2 and 100% by weight with respect to the weight of hexose.

11. Process according to any one of claims 1 to 10, for which the quantity of hexose is between 0.5% and 35% by weight with respect to the total water + carboxylic acid weight.

12. Process according to any one of claims 1 to 9, for which the pH at the start of the reaction is between 1 and 3.

13. Process according to any one of claims 1 to 11, conducted at a temperature of between 100 and 200°C.

14. Process according to any one of claims 1 to 13, conducted in the absence of organic solvent.

15. Process according to any one of claims 1 to 14, conducted continuously.
